# EUROPEAN PATENT SPECIFICATION

(11) **EP 3 277 282 B1**
(45) Date of publication and mention of the grant of the patent: **12.10.2022**
(21) Application number: 16728423.1
(22) Date of filing: 01.04.2016
(51) Int. Cl.: A61K 31/506, A61K 9/00, A61K 45/06, A61P 11/00

(54) **DASATINIB FOR USE IN THE TREATMENT, REGRESSION OR DECELERATING THE PROGRESS OF FIBROTIC LUNG DISEASES**
DASATINIB ZUR VERWENDUNG IN DER BEHANDLUNG, REGRESSION ODER VERZÖGERUNG DES FORTSCHRITTS VON FIBROTISCHER LUNGENERKRANKUNGEN
UTILISATION DE DASATINIB POUR LE TRAITEMENT, LA RÉGRESSION OU LE RALENTISSEMENT DE LA PROGRESSION DE MALADIES PULMONAIRES FIBROTIQUES

(30) Priority: 01.04.2015 TR 201503969
(43) Date of publication of application: 07.02.2018
(73) Proprietor: Istanbul Universitesi Rektorlugu, 34452 Istanbul (TR)
(72) Inventor: OZTAY, Fusun, 34119 Istanbul (TR); YILMAZ, Oznur, 34119 Istanbul (TR)
(74) Representative: Kaya, Erdem
(86) International application number: PCT/TR2016/050095
(87) International publication number: WO 2016/159917

(56) References cited:
- Erin Gurley: "Dasatinib inhibits alpha-sma assembly in myofibroblasts differentiation: a potential role in pulmonary fibrosis", Pharmacotherapy, Volume 33, Number 10, 1 October 2013 (2013-10-01), page e293, XP055299593, Retrieved from the Internet: URL:https://www.accp.com/docs/meetings/abs tracts/2013_annual.pdf [retrieved on 2016-09-02]
- Leeann Thompson: "Dasatinib, a Src inhibitor, attenuates alpha-SMA synthesis: role in pulmonary fibrosis", Pharmacotherapy, Volume 33, Number 10, 1 October 2013 (2013-10-01), pages e293-e294, XP055299584, Retrieved from the Internet: URL:https://www.accp.com/docs/meetings/abs tracts/2013_annual.pdf [retrieved on 2016-09-02]
- DATABASE MEDLINE [Online] US NATIONAL LIBRARY OF MEDICINE (NLM), BETHESDA, MD, US; 2015, YILMAZ OZNUR ET AL: "Dasatinib attenuated bleomycin-induced pulmonary fibrosis in mice.", XP002761432, Database accession no. NLM26607773 & GROWTH FACTORS (CHUR, SWITZERLAND) 2015, vol. 33, no. 5-6, 2015, pages 366-375, ISSN: 1029-2292
- I. A. DIMITROULIS: "Nintedanib: A Novel Therapeutic Approach for Idiopathic Pulmonary Fibrosis", RESPIRATORY CARE, vol. 59, no. 9, 29 April 2014 (2014-04-29) , pages 1450-1455, XP055137430, ISSN: 0020-1324, DOI: 10.4187/respcare.03023
- Anonymous: "Pirfenidone: ANNEX I SUMMARY OF PRODUCT CHARACTERISTICS", , 28 February 2011 (2011-02-28), XP055679110, Retrieved from the Internet: URL:https://www.ema.europa.eu/en/documents /product-information/esbriet-epar-product- information_en.pdf [retrieved on 2020-03-24]
- Anonymous: "Nintedanib: ANNEX I SUMMARY OF PRODUCT CHARACTERISTICS", , 15 January 2015 (2015-01-15), XP055679106, Retrieved from the Internet: URL:https://www.ema.europa.eu/en/documents /product-information/ofev-epar-product-inf ormation_en.pdf [retrieved on 2020-03-24]
- Anonymous: "Pulmonary fibrosis - Diagnosis and treatment - Mayo Clinic", , 6 March 2018 (2018-03-06), XP055679120, Retrieved from the Internet: URL:https://www.mayoclinic.org/diseases-co nditions/pulmonary-fibrosis/diagnosis-trea tment/drc-20353695 [retrieved on 2020-03-24]

## Description

### FIELD OF THE INVENTION

The present invention is defined by the claims. The present invention relates to the use of dasatinib or its pharmaceutically acceptable salt or solvate thereof as a pharmaceutical agent for regression or slowing the progression of fibrosis in fibrotic lung diseases, in particular idiopathic pulmonary fibrosis disease.

### BACKGROUND OF THE INVENTION

Fibroblasts and myofibroblasts are target cells of a variety factors including platelet-derived growing factor (PDGF) and fibroblast growing factor (FGF), connective tissue growth factor (CTGF) and epidermal growth factor. Growth factors show their effect on target cells through receptor tyrosine kinases (RTKs) on cell membrane. Dimerization formed by ligand effect and activation of RTK subunits stimulates the tyrosine kinase activity of the receptor(phosphorylation of tyrosine residues of proteins) and it triggers the extracellular signal-regulated kinases (ERK1/2) and signaling pathway of serine/threonine kinase-protein kinase B (Akt). Over stimulation of intracellular signaling pathways, with the effect of proliferation, migration and differentiation of the cells of various types such as fibroblasts, myofibroblasts, high expression of collagens and accumulation in the interstitial area causes pulmoner fibrosis (Antoniu, 2012). Abnormal fibroblast proliferation and excess myofibroblast differentiation are important indications of the development and progression of pulmonary fibrosis. Fibroblast proliferation and myofibroblast differentiation are under the control of PDGF, FGF, CTGF (transforming growth factor-beta)TGF-β and various cytokins (Wynn, 2008; Bonner, 2010). Fibroblasts expressing PDGF receptor-alpha (PDGFR-a). This receptor is located on the fibroblast cell surface membrane.In addition, PDGF induced fibroblast proliferation, fibroblast/myofibroblast migration and collagen and fibronectin production are observed in pulmonary fibrosis.

Anti-fibrotic therapies that are selectively targeting the activation of increasing production of extracellular matrix proteins and fibroblasts for pulmonary fibrosis diseases are not available yet.

Various studies provide strong data showing some RTK antagonists and inhibitors induce regression of pulmonary fibrosis in rodent bleomycin (BLM) models. The compounds used in these studies block the fibrotic signals through the inactivation of RTK of PDGF, FGF and vascular endothelial growth factor (VEGF) and then show anti-fibrotic effects (Ou et al., 2009; Adachi et al., 2010; Antoniu, 2012). However, the majority of them have not been tested yet in clinical trials of pulmonary fibrosis diseases. Among them, imatinib and nintedanib were evaluated as potential idiopathic pulmonary fibrosis therapies (Daniels et al., 2010; Richeldi et al., 2011). Despite the promising results of these studies, the use of RTK inhibitors in the treatment of pulmonary fibrosis is controversial.

RTK inhibitors have some side effects such as pulmonary toxicity in human, diffuse alveolar damage, acute interstitial pneumonia and increase in liver enzyme levels (Barber and Ganti, 2011; Iwomoto et al., 2011). For this reason,a great effort is given during ongoing studies for the definition of an effective and reliable RTK inhibitor for the treatment of pulmonary fibrosis. Dasatinib is an RTK inhibitor, the generic name of N-(2-chloro-6-methylphenyl)-2-[[6-[4-(2-hydroxyethyl)-1-piperazinyl]-2-methyl]-4-pyrimidinyl]amino]-5-thiazolecarboxamide mono-hydrate compound,and represented as below Formula (I), also known as BMS-354825 and SPRYCEL^{™}.

Dasatinib is soluble is ethanol, methanol and organic solvents.

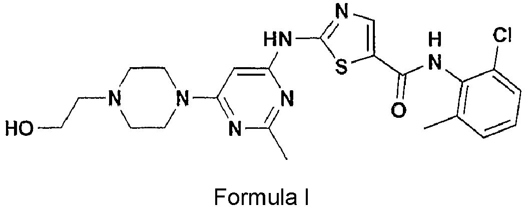

Dasatinib is widely used for therapeutic purposes in some types of cancer and chronic myeloid leukemia patients due to its antiangiogenic and anti-proliferative properties (Johnson et al., 2005; Montero et al., 2011).

WO 2007/047893 A2 discloses the use of dasatinib in the treatment of bone metastasis.

WO 2007/047893 A2 discloses the use of dasatinib for treating or inhibiting of restenosis and stenosis.

Also, it has been reported that dasatinib lowers mRNA and protein levels of extracellular matrix proteins in the stimulated dermal fibroblasts, which have been isolated from patients that has sklerodermas. In addition, there are evidences that dasatinib decreases the number of myofibroblast and skin collagen content in the BLM sourced dermal fibrosis(Akhmedshina et al., 2008).

In the present invention, it has been found that dasatinib, as an RTK inhibitor, surprisingly slows the progression of the pulmonary fibrosis by reducing fibroblast proliferation, myofibroblast differentiation and interstitial collagen deposition in fibrotic lung diseases, in particular idiopathic pulmonary fibrosis disease GURLEY, E. "Dasatinib inhibits alpha-sma assembly in myofibroblasts differentiation: a potential role in pulmonary fibrosis". Pharmacotherapy, Volume 33, Number 10, 1 October 2013, page e293, reports a potential role of dasatinib in pulmonary fibrosis by inhibiting alpha-sma assembly in myofibroblasts differentiation.

THOMPSON, L., "Dasatinip, a Src inhibitor, attenuates alpha-SMA synthesis: role in pulmonary fibrosis", Pharmacotherapy, Volume 33, Number 10, 1 October 2013, page e293- e294, reports a role of dasatinib in pulmonary fibrosis as a Src inhibitor, attenuating alpha-SMA synthesis.

Both documents merely disclose a potential activity of dasatinib in lung fibrosis with in-vitro evidence. None of them disclose a credible demonstration of therapeutic activity of dasatinib fibrotic lung diseases.

### SUMMARY OF THE INVENTION

The present invention is defined by the claims.

Thus, the present invention relates to a compound of Formula (I) as depicted in claim 1 or its pharmaceutically acceptable salts thereof, for treatment, regression or slowing the progression of fibrotic lung disease wherein the composition is administered through at least one route selected from inhalation or pulmonary oscillation.

### BRIEF DESCRIPTION OF THE DRAWINGS

Figure 1. Lung histology sections of mice stained with hematoxyline-eosin or Masson's trichrome stain. In mice treated with BLM, accumulated collagen fibers in the extracellular matrix, stained with Masson trichome stain of lung sections are shown as green (Scale bar 100 µm). The alveoli (A), the cells into the alveolar lumen (arrowheads), fibrotic focus (F), the mono-nuclear cell infiltration in the fibrotic focus (asterix), terminal bronchial (TB) and the vein (V). The scale is 200 µm.
Figure 2. Ashcroft score is shown in the experimental groups. The results give ± SD. *p < 0.05 results as average values.
Figure 3. In the experimental groups nuclear localization of anti-Ki67 immunoreactivity is demonstrated by arrow (a). In negative control staining immunoreactivity has not been determined. Fibrotic foci (F). Scale = 100 m. Mayer's hematoxyline stain.
In Figure 3b, the number of proliferating cells in the alveolar regions (%) is shown. The results represent the average value ± SD **p < 0.01 compared to the control group.***p < 0.001 is shown against BLM group.

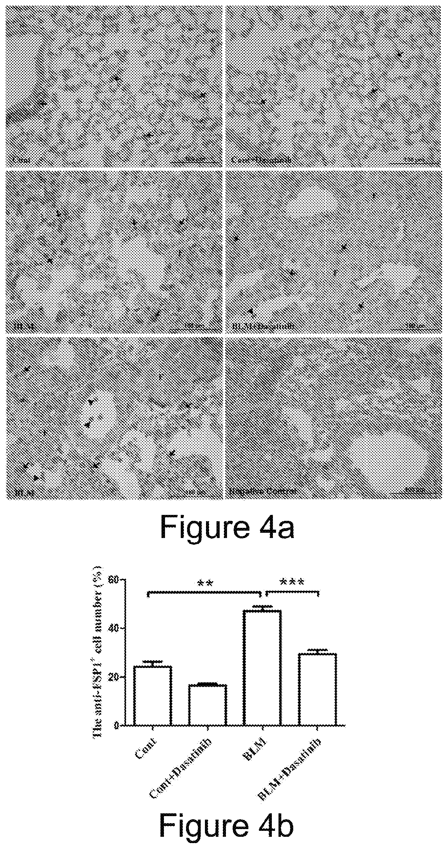

Figure 4. Cytoplasmic localization of anti-FSP1 immunoreactivity is shown by an arrow (a) in the lung of the mice. In negative control staining immunoreactivity has not been determined.Fibrotic foci (F). Scale = 100 m. Mayer hematoxyline contrast staining.In addition, in the cytoplasm of fibroblast (arrowhead) in the alveolar lumen (rarely in the bronchial lumen) of mice lungs treated with BLM, anti-FSP1 immunoreactivity is available. In Figure 4b, the number of fibroblasts (%) in the experimental groups is shown. Dasatinib treatment resulted in a decrease in the number of fibroblasts in the mice lungs treated with BLM. The results represent the average value ± SD. *p < 0.05 compared to the control group. Against control group, **p < 0.01. Against BLM group, ***p < 0.001.
Figure 5. Typical protein bands in the experimental groups (a) and collagen-1 (b) levels. Values are presented as mean values ± SD (per group, n = 5). *p < 0.05.
Figure 6. p-PDGFR-α and -β (b) levels and typical protein bands in the experimental groups (a). Values are presented as mean values ± SD (per group, n = 5). *p < 0.05.
Figure 7. p-Akt, p-ERK1/2, PTEN and p-c-Abl (b) levels and typical protein bands in the experimental groups (a). Values are presented as mean values ± SD (per group, n = 5). *p < 0.05, **p < 0.01.

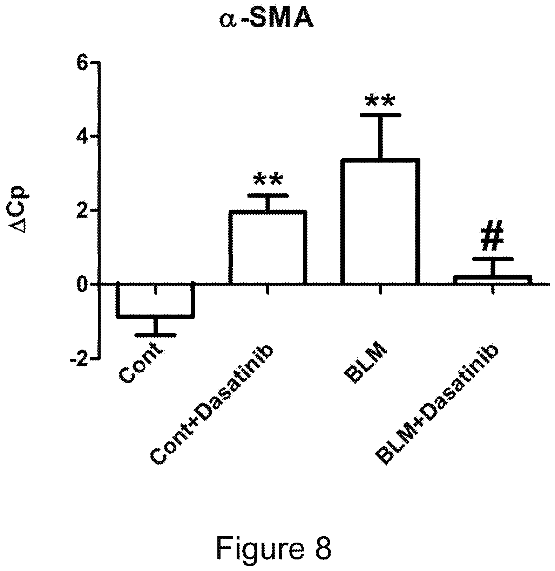

Figure 8. Data show the mRNA levels of the α-SMA gene in the experimental groups. Cp: cycle number of crossing point. Relative expression levels, ΔCp = Cp reference - Cp is calculated as a target. Against control group, **p < 0.01. Against BLM group, #p < 0.001.

### DETAILED DESCRIPTION OF THE INVENTION

One aspect of the present invention relates to a compound of Formula (I) or its pharmaceutically acceptable salts thereof, for use in treatment, regression or slowing the progression of fibrotic lung disease wherein the compound is administered through at least one route selected from inhalation or pulmonary oscillation.

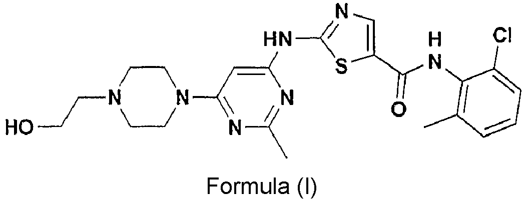

In one embodiment of the present invention, the compound is administered through the pulmonary oscillation route.

In another embodiment of the present invention, the compound is administered in a daily dose range of 0.05-300 mg.

In another embodiment of the present invention, said fibrotic lung disease is idiopathic pulmonary fibrosis observed in human.

A specific compound can be found in one or more particular geometric, optical, enantiomeric, diasteriomeric, stereoisomeric and conformational forms. The compound of the present invention can also be in a pharmaceutical acceptable salt form, because it may be appropriate to prepare, purify and use the corresponding salt. The compounds may be in a hydrate form, because it may be appropriate to prepare, purify and use the relevant hydrate form e.g., mono-hydrate, di-hydrate etc. The compounds may also be in a "chemically protected" form because it may be appropriate to prepare, purify and use the relevant protected form. "Chemically protected form" means a compound that contains one or more chemically active functional group and protected from chemical reactions with the help of a protective group.

### The Use of the Compounds

The subject compounds of the present invention used as active compounds having anti-fibrotic and/or anti-proliferative activity, for the treatment, regression or slowing the progression of fibrotic lung diseases in particular idiopathic pulmonary fibrosis. Here, the term "active" used for anti-fibrotic activity, relates to the compounds that can regulate the formation of fibroblast in the cell. Here, the term "active" used for anti-proliferative activity, relates to the compounds that can regulate cell proliferation.

The subject compounds of the present invention, used as anti-fibrotic and/or anti-proliferative agents, for the treatment, regression or slowing the progression of fibrotic lung diseases in particular idiopathic pulmonary fibrosis. Here, the term "anti-fibrotic agent" relates to a compound that treats a fibrotic situation. Here, the term "anti-proliferative agent" relates to a compound that treats a proliferative situation.

The present invention also provides active compounds for use in a method for treatment of the human or animal body. Such a method includes the implementation of a pharmaceutically effective amount of an active compound to the subject, preferably in the form of a pharmaceutical composition.

The term "treatment" used here as a treatment of a disease relates generally to the treatment and therapy where some desired pharmaceutical effects are derived.

The term "pharmaceutically effective amount" used here relates the amount of the active compound or a composition that contains an active compound effective to bring a portion of the desired pharmaceutical effect or the amount of dosage form.

The compound for use according to the present invention can be applied to a subject in need of treatment, in combination with various antioxidants, anti-inflammatory and anti-fibrotic agents such as TGF-β, PDGF, EGF, FGF, VEGF, CTGF, endothelin inhibitor, antibody and antagonists, collagenase activators and N-acetyl cysteine.

### Application

The active compound or the pharmaceutical formulation containing active compound which is the subject of the present invention, systemically can be applied in a region, including, but not limited to, e.g., subcutaneous, intraperitoneal, intramuscular and intravenous injection or pulmonary e.g., by inhalation or the through the pulmonary oscillation route or, oral or parenteral. Only inhalation and pulmonary oscillation represent routes according to the invention.

On the other hand, dasatinib is given to human by oral route and known to cause some systemic side effects such as bleeding in the intestines. Therefore, according to the invention dasatinib is given to the subject by inhalation or by the ways of oscillation to the lungs.

### Formulations

The active compound of the present invention is preferred to be formulated as a pharmaceutical composition that includes at least one active component and one or more pharmaceutical acceptable additives such as carrier, filler, buffer, adjuvant, stabilizer or other materials.

The term "pharmaceutically acceptable" relates to compounds, materials or components that are suitable for use in contact with tissue of a subject without causing excessive toxicity, irritation, allergic response or any other problems.

Formulations can be prepared by any known method in the pharmaceutical literature and can be presented in unit dosage form. Formulations, can be -but not limited to- in the form of tablet, capsule, syrup, lozenge, pill, capsule, sachet, ointment, gel, cream, spray, paste, aerosol or in the form suppository.

### Experiments

### Materials and methods

### Animals

**Adult male** C57BL/6J mice (10-12 weeks of age, 20-30 g of weight) are housed in a temperature and humidity controlled rooms in 12 hours light/dark cycle and were given food and *ad libitum* water.

### Experimental Procedures

Mice were divided into such experimental groups (n = 10 per group): after a single dose of BLM instillation (0.08 mg/kg in 200 µl sterile physiological saline solution) the mice examined on the first 21st day (I) and their controls (II), after a single dose of BLM instillation for 1 week treated with dasatinib following 14 days (8 mg/kg in 100 µl with gavage) and slaughtered mice on the 21st day of the experiment (III)and their controls (IV).

Pulmonary fibrosis in mice was induced with BLM under 20 mg/kg ketamine anesthesia. In a BLM 200 µl sterile physiological saline solution 0.08 mg/kg was administered as a single intra-tracheal dose (Obed and Öztay, 2014). Dasatinib was dissolved in a mixture of 1:9 dimethyl sulfoxide/sterile physiological saline solution. Mice were killed for analysis on the 21st day of the experiment before lungs collection by a 1:1 mix ratio 40 mg/ketamine and 10 mg/kg intraperitoneal xylazine injection.The lungs of five mice from each group were used for microscopic analysis, while the remaining five mice lungs were collected for immunoblotting analysis and quantitative real-time polymerase chain reaction (qRT-PCR).

### Reagents and antibodies

Reagents and antibodies were obtained from the following sources: BLM from Neon Laboratories (Mumbai, India); ketamine from Pfizer (Kirklareli, Turkey); xylazine from Alfasan International BV (Woerden, Netherlands); dasatinib, radioimmunoprecipitation experiment buffer, luminal reagent, antiphosphorylated (p)- Akt1/2/3 antiphosphotase and chromosome 10 tensin homolog (PTEN) and anti-beta(β)actin primer antibodies and goat anti-rabbit seconder antibody from Santa Cruz (Santa Cruz, CA, USA); histostatin plus kit and quant-it protein assay kit from Invitrogen (Paisley, Scotland); 3-amino-9-ethyl carbazol substrate kit from Thermo Scientific (Waltham, MA, USA); total RNA purification kit from Peqlab (Salisbury Green, England); complementary DNA (cDNA) synthesis kit from Roche (Grenzach-Wyhlen, Germany); anti-Ki67 and anti-p-PDGF receptor subunit alpha (p-PDGFR-a) antibodies from Millipore (Molsheim, France); anti-fibroblast specific protein 1 (FSP1) from Abcam (Cambridge, UK); anticollagen-1 antibody from Novus Biologicals (Littleton, CO, USA); anti-p-PDGFR-β antibody from Upstate Biotechnology Inc. (New York, USA); anti-p-cytoplasmic Abelson kinase (P-C-Abl) antibody from Bioss (Woburn, Massachusetts, USA); anti-pERK1/2 antibody from Cell Signaling Technology (Danvers, MA, USA).

### Histology and Immunohistochemistry (each group n = 5)

The lungs were identified with 10% natural buffered formalin and then were kept in the same solution for 24 hours at 4°C. These were processed and divided into 4-µm-thick serial sections. One section was chosen in every twenty-five sections (at intervals of 100 µm) and placed on slides. Sections were used for hematoxyline-eosin and Masson's trichromo staining and immuno labeling. These were evaluated under a light microscopy (Nikon Eclipse Ti-U, Tokyo, Japan) equipped with a digital camera (Nikon LH-M100C-1 Camera, Tokyo, Japan).

The degree of fibrotic changes in mouse lungs, 42 randomly selected microscopic fields at x100 magnification (non-bronchial alveolar spaces) per mouse lung, through the analysis of modified the size of the Ashcroft fibrosis (0 to 8):scores 0 and 1represented absence of fibrosis, scores 2 and 3 represented minimum fibrosis, scores 4 and 5 were accepted to represent moderate fibrosis and scores 6 and 8 represented severe fibrosis.

For immunohistochemistry streptavidin-biotin immuno-enzymatic antigen diagnostic system was used, parts were treated for 15 minutes in a 650 W microwave oven in pH 9.0 10 mM tris-EDTA buffer. Endogenous peroxidase was eliminated through incubation in 1:1 methanol/phosphate-buffered saline mixture with 3% hydrogen peroxide. Sections were treated with a rabbit anti-Ki67 antibody (proliferation indicator) in 1:200 dilutions at room temperature for 1 hour and with a rabbit anti-FSP (fibroblast indicator) in 1:100 dilutions at 4°C overnight.

Later, sections were incubated in a humidified chamber according to manufacturer's instructions in the Histostain-Plus-peroxidase kit. Peroxidase activity was shown through 3-amino-9-ethylcarbazole substrate kit. Slides are stained gently again with Mayer's hematoxyline to reveal cell nuclei. For negative control, phosphate buffered saline solution was used instead of primary antibodies.

In each of the sections randomly selected from each mouse, five microscopic fields were randomly selected from non-bronchial alveolar field. Digital images of these fields were taken at x400 magnification, and overlapped with the transparent grids (1×1 mm). Then, proliferative cells in adjacent sections and fibroblasts werecounted. The cell proliferation index and number of fibroblasts were calculated as the percentage of immuno-reactive cells in the total cell number.

### Western blot analysis (each group n = 5)

The lungs suddenly cooled in liquid nitrogen and were kept at -86 °C. The lungs were homogenized in cold radio-immunoprecipitation analysis buffer containing 1mm phenylmethylsulfonyl fluoride,0.2 IU/ml aprotinin and 1 mM sodium orthovanadate. Then, lysates were centrifuged at 10.000 g for 10 minutes at 4 °C. The total protein level in the lungs was determined using a Quant-it protein analysis kit. Samples (80 µg protein) were mixed with sample buffer, denaturated by heating for 5 minutes at 95 °C, decomposed with SDS-PAGE and transferred to nitrocellulose membranes. Membranes were blocked for 1 hour with 5% nonfat dried milk in tris-buffered saline (TTBS) with tween andwere incubated all over night at 4°C against primary antibodies and collagen-1 (1:1,000 dilution), activated p-PDGFR-a (1:100 dilution), p-PDGFR-β (1:200 dilution), p-c-Abl (1:200 dilution), p-ERK1/2 (1:1,000 diluted), p-Akt1/2/3 (1:100 dilution) and PTEN ( ERK/MAPK an inhibitor protein) and PI3/Akt (1:500 dilution).Later, these were washed with TTBS and incubated for 1 hour in 1:5.000 dilutions with horseradish peroxidase conjugated goat anti-rabbit. Finally, blots were developed with luminal reagent. The intensity of protein bands was measured by using Kodak Gel Logic Molecular Imaging software (Kodak, GL 1500, Connecticut, USA) and normalized to β-actin protein bands (1:500 dilution). Values were presented as multiple increases in protein expression.

### Tissue Total RNA Isolation and qRT-PCR (each group n = 5)

Total RNA was isolated from lungs using PeqGold total RNA purification kit and then DNaz was treated to remove contaminated genomic DNA. cDNA was synthesized from 1 µg total RNA with cDNA synthesis kit. Total RNA were scanned to demonstrate equivalence of RNA, using primers (forward, 5'CTCTCTTCCAGCCATCTTTCAT3'; back, 5'TATAGGTGGTTTCGTGGATGC3') and β-actin gene (forward, 5'CTAAGGCCAACCGTGAAAAG3'; back, 5'ACCAGAGGCATACAGGGACA3'),for mRNA encoding alpha-smooth muscle actin (a-SMA, miyofibroblast indicator).According to the manufacturer's instructions qRT-PCR was carried out for all primer/probe and template combinations.qRT-PCR analysis was performed by following the method described in previous studies (Kayalar and Oztay, 2014). As internal control, a standard curve was created for quantification of desired gene by using β-actin. The relative cDNA ratio was calculated using the threshold cycles value.Relative expression levels were calculated using Light Cycler 480 Analysis System (Roche, Mannheim, Germany), ΔCt=Ct ^{reference} - Ct ^{target}.

### Statistical Analysis Data

Statistical analysis was evaluated using Graphpad 5.0 software (San Diego, USA). These were performed by non-parametric Mann-Whitney U test following one-way ANOVA. Values were explained as mean ± standard deviation (SD). Significance set at p < 0.05.

### Results

### Example 1

### Histological results

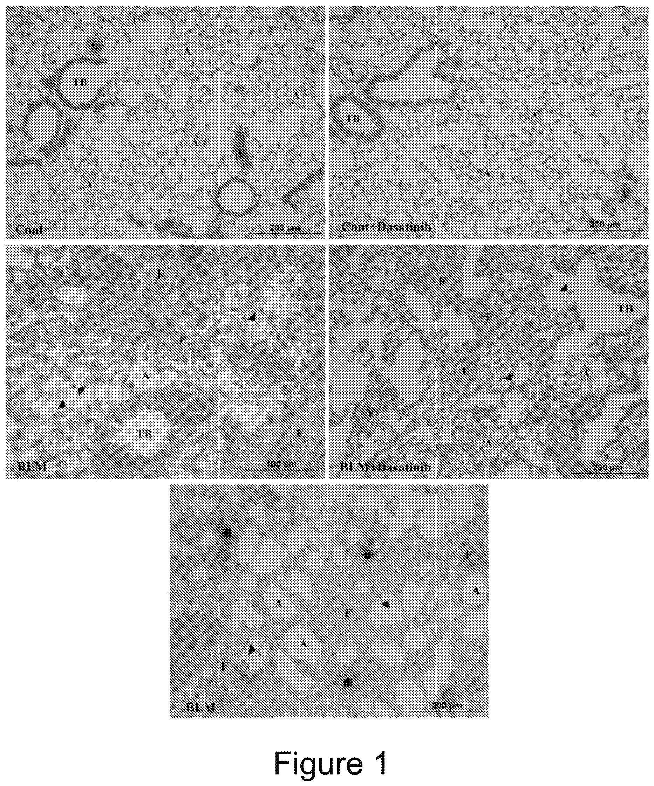

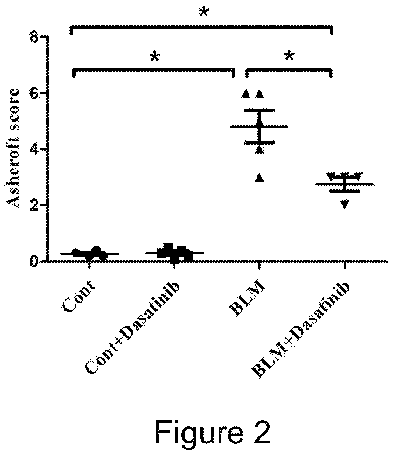

Control mice taken vehicle and dasatinib exhibited a healthy lung structure (Figure 1).Intra-tracheal injection of BLM caused destruction of normal pulmonary architecture and an increase in mesenchymal cell stack parts, infiltration of inflammatory cells and formation of fibrotic fociwith the extensive accumulation of fibrillary collagen. Additionally, some cells were observed in the alveolar lumen in the lungs of BLM applied mice (Figure 1). In the lungs of the BLM treated mice the degree of fibrosis showed variability between 3 and 7 (Figure 2).In general, the structure of the damaged lung of mice produced confluent fibrotic masses (>10%and ≤50%microscopic spaces).Alveoli were rarely observed in the large adjacent fibrous masses. Dasatinib was able to reduce the severity of lung damage and interstitial collagen fibers and decreased the degree of fibrosis in the lungs of BLM treated mice, on average, from 5 to 3 (Figure 2). Also, treatment with dasatinib has reduced the prevalence of fibrotic areas in the lungs of BLM treated mice and in general single fibrotic mass was produced instead of large adjacent fibrotic masses.

### Example 2

### Anti-Ki67 and anti-FSP1 immunoreactivities

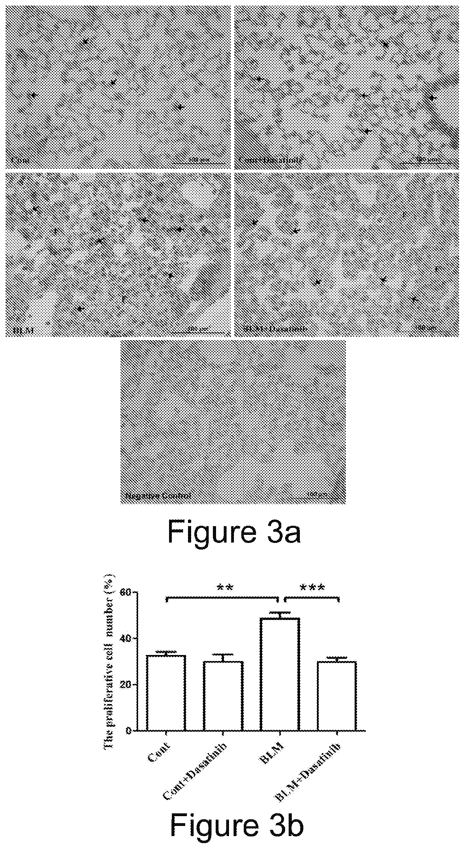

Anti-FSP1 immunoreactivity was observed in the cytoplasm of fibroblasts in the lung while on the nuclei of epithelial and mesenchymal cells anti-Ki67 immunoreactivity was observed.Mice treated with BLM, when compared with control mice, exhibited a significant increase in the number of fibroblasts and cell proliferation, especially in fibrotic foci(Figure 3 a, b and Figure 4 a, b; p<0.01).These changes were consistent with the degree of fibrosis increased observed in the lungs of mice treated with BLM. Additionally, anti-FSP1 was found in the cytoplasm of some cells in in the alveolar lumen of the lungs of mice treated with BLM. Dasatinib treatment caused a decrease in cell proliferation in the alveolar space of the lungs of mice treated with BLM and in the number of fibroblasts (Figure 3 a, b and Figure 4 a, b; p < 0.01).In addition, in the lungs of mice treated with dasatinib, the decrease in the number of proliferating cells in large fibrotic fociand in the number of fibroblasts were remarkable.

### Example 3

### Western blotting

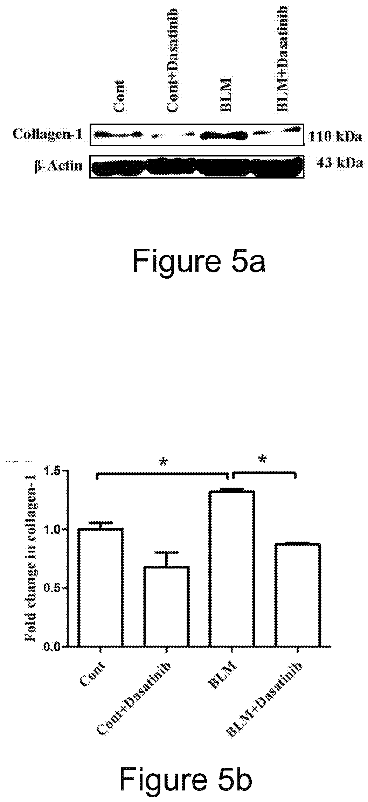

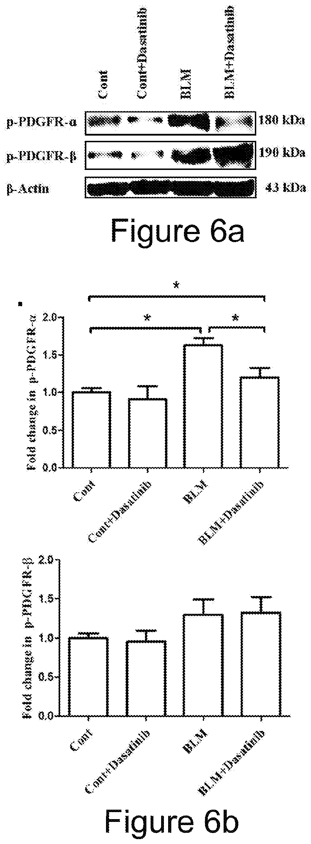

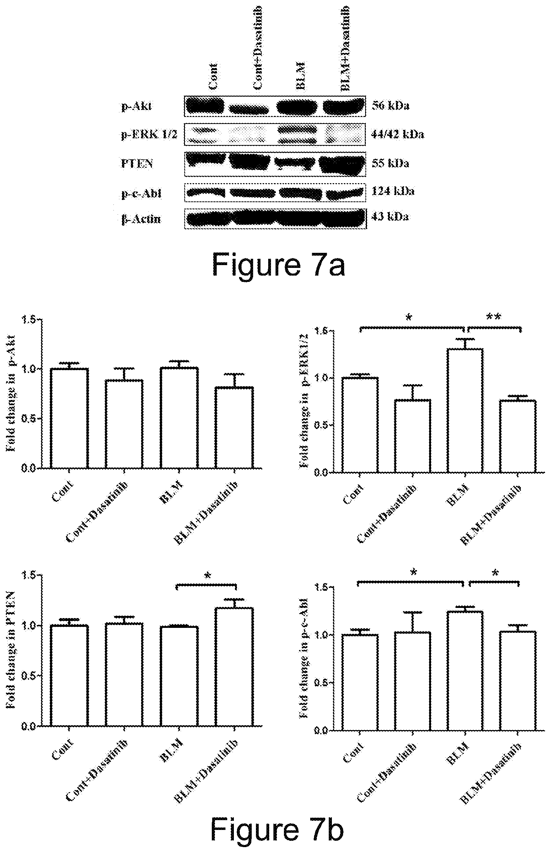

BLM treatment, caused a significant increase in collagen-1, p-PDGFR-a, p-c-Abl and pERK1/2 expression levels, when compared with those obtained in control mice (Figure 5, 6 and 7; p< 0.05). However, the decrease in PTEN expression observed in the lungs of these mice and the increase in the level of expression of p-PDGFR-β and p-Akt1/2/3 was not significant (Figure 6 and Figure 7).High expression of collagen-1, p-PDGFR-a, p-c-Abl and pERK1/2 was reduced by treatment of dasatinib and PTEN expression was significantly stimulated in the lungs of micetreated with BLM (Figure 5, Figure 6 and Figure 7; p< 0.05). The decrease in the p-ERK1/2 expression was more significant in fibrotic lungs after treatment of dasatinib (p< 0.01).

### Example 4

### qRT-PCR

The level of mRNA of α-SMA, was induced with BLM in the lungs of mice. Dasatinib treatment, diminished the increase in the level of mRNA of α-SMA in the lungs of mice treated with BML.

Accordingly, with the present invention, it was found that dasatinib has weakened PDF-induced pulmonary fibrogenesis of the lungs of mice treated with BLM via selective inhibition of PDGFR-a activation.In mouse lungs treated with dasatinib, the aforesaid invention isconfirmed by the decrease in observed p-PDGFR-α levels even though fibrosis is in a chronic stage, fibrosis degree, collagen-1 mRNA and protein expression levels and the collagen accumulation.Studies on pulmonary fibrosis, respectively, FSP1 and α-SMA, fibroblast and myofibroblast are unique identifiers. In the primary lung cell cultures and C57BL mouse lungs, while macrophages do not express FSP1 protein, fibroblasts express FSP1 (Lawson et al., 2005).Based on these findings, it is possible to say that FSP1-immuno-reactive cells found in fibrotic foci and alveolar lumen are the fibroblasts in lung of mice treated with BLM.In the studies made for the present invention, the number of FSP1-immuno-reactive cells in the lungs after BLM treatment, has increased in line with the findings of other studies performed on rodent BLM models. In addition, increased number of fibroblast were accompanied with an increase in the number of cell proliferation in alveolar area in these mice, the rising of α-SMA mRNA and collagen-1 mRNA/protein levels and fibrotic areas spread throughout lung tissue.

In the lungs of mice treated with BLM, less fibroblast was observed in the alveolar lumen after treatment of dasatinib. Therefore dasatinib, in the therapy of pulmonary fibrosis, inhibits formation of new fibrotic areas and the spread of existing fibrotic foci and dasatinib treatment was found to be reliable for pulmonary fibrosis and is a binary tyrosine kinase inhibitor.

## Claims

1. A compound of Formula (I) or its pharmaceutically acceptable salts thereof, for use in treatment, regression or slowing the progression of fibrotic lung disease wherein the compound is administered through at least one route selected from inhalation or pulmonary oscillation.

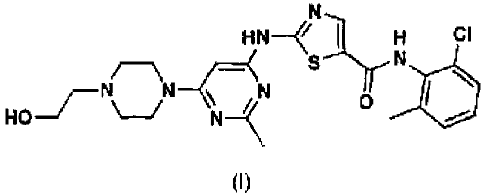

2. A compound for use according to claim 1, wherein the compound is administered through the pulmonary oscillation route.

3. A compound for use according to claim 1 or claim 2 , wherein the compound is administered in a daily dose range of 0.05-300 mg.

4. A compound for use according to Claim 1, wherein said fibrotic lung disease is idiopathic pulmonary fibrosis observed in human.

## Patentansprüche

1. Verbindung der Formel (I) oder pharmazeutisch verträgliche Salze davon zur Verwendung bei der Behandlung, Rückbildung oder Verlangsamung des Fortschreitens einer fibrotischen Lungenerkrankung, wobei die Verbindung über mindestens einen Weg verabreicht wird, der aus Inhalation oder pulmonaler Oszillation ausgewählt ist..

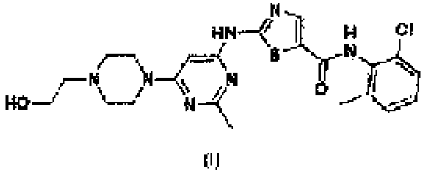

2. Verbindung zur Verwendung nach Anspruch 1, wobei die Verbindung über den Weg der pulmonalen Oszillation verabreicht wird.

3. Verbindung zur Verwendung nach Anspruch 1 oder Anspruch 2, wobei die Verbindung in einem Tagesdosisbereich von 0.05-300 mg verabreicht wird.

4. Verbindung zur Verwendung nach Anspruch 1, wobei es sich bei der fibrotischen Lungenerkrankung um eine beim Menschen beobachtete idiopathische Lungenfibrose handelt.

## Revendications

1. Composé de Formule (I) ou ses sels pharmaceutiquement acceptables, destiné à être utilisé dans le traitement, la régression ou le ralentissement de la progression d'une maladie pulmonaire fibrotique, dans lequel le composé est administré par au moins une voie choisie parmi l'inhalation ou l'oscillation pulmonaire.

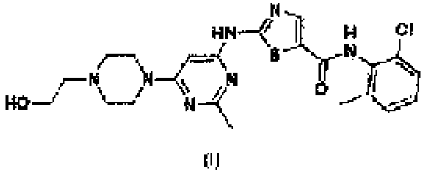

2. Composé destiné à être utilisé selon la revendication 1, dans lequel le composé est administré par la voie de l'oscillation pulmonaire.

3. Composé destiné à être utilisé selon la revendication 1 ou la revendication 2, dans lequel le composé est administré à une dose quotidienne comprise entre 0.05 - 300 mg.

4. Composé destiné à être utilisé selon la revendication 1, dans lequel ladite maladie pulmonaire fibrotique est une fibrose pulmonaire idiopathique observée chez l'homme.
